# EUROPEAN PATENT APPLICATION

(11) **EP 3 711 765 A1**
(43) Date of publication of application: **23.09.2020**
(21) Application number: 18896151.0
(22) Date of filing: 10.10.2018
(51) Int. Cl.: A61K 31/52, A61K 31/352, A61K 36/185, A61K 9/20, A61K 9/14, A61K 9/08, A61K 9/06, A23L 2/00, A23L 7/00, A61P 25/22

(54) **COMPOSITION CONTAINING CANNABIDIOL/CANNABIS EXTRACT AND CAFFEINE, AND APPLICATION OF COMPOSITION**

(30) Priority: 29.12.2017 CN 201711471435
(71) Applicant: Hanyi Bio-Technology (Beijing) Co., Ltd., Beijing 100020 (CN)
(72) Inventor: ZHANG, Ke, Beijing 100020 (CN); TAN, Xin, Beijing 100020 (CN); YU, Zhaohui, Beijing 100020 (CN); CHANG, Tanran, Beijing 100020 (CN); LIAN, Meng, Beijing 100020 (CN); JIN, Qian, Beijing 100020 (CN)
(74) Representative: Hanna Moore + Curley
(86) International application number: PCT/CN2018/109658
(87) International publication number: WO 2019/128377

(57) **Abstract**

The invention provides a composition comprising cannabidiol or a cannabis extract, and caffein. The mass percentage of the cannabidiol in the cannabis extract is 10%-99%. A mass ratio of the cannabidiol to the caffein in the composition is (1-100):60. The composition can be a food composition or a pharmaceutical composition. The cannabidiol or the cannabis extract can prevent and/or ameliorate adverse reactions induced by the caffeine, and in particular, prevent and/or ameliorate anxiety behaviors induced by the caffeine.

## Description

### BACKGROUND OF THE INVENTION

### 1. Technical Field

The invention relates to the field of medicine and health, and in particular to a composition containing cannabidiol or a cannabis extract and caffeine, and application thereof in the preparation of food or medicines.

### 2. Description of Related Art

Caffeine is a methylxanthine alkaloid that can stimulate the heart, the skeletal muscles and the central nervous system, and has the effects of promoting gastric acid secretion and diuresis and treating diseases such as migraine. The physiological activity mechanism of the caffeine is largely related to promoting the intracellular release of Ca²⁺ and increasing the antagonistic action of intracellular cAMP concentration against adenosine receptors A1 and A2A. The long-term chronic or single large-dose (> 400mg) intake of the caffeine may cause a series of physical and psychological adverse reactions, such as excessive excitement, nervousness, restlessness, insomnia, palpitations, tremor, brain metabolic disorders, etc.

Cannabidiol (CBD) is a main chemical component present in cannabis as a pharmaceutical plant. It is extracted from the cannabis plant as a non-addictive component in cannabis. The CBD itself has an effect of agonizing the adenosine receptor A2A. The CBD plays roles such as sedation, anticonvulsion, and anti-anxiety in the central nervous system; and in the cardiovascular system, the CBD can vasodilate blood vessels and reduce myocardial contractility by inhibiting L-type Ca²⁺ channels.

Patent US20160250270A1 discloses a beverage composition, which may contain 0.1-10 wt% of CBD and 0.015-15 wt% of caffeine.

Patent US20160324776A1 discloses a caffeine-containing beverage containing cannabinoid, wherein the beverage retains its original flavor and appearance; and the cannabinoid may have the content of 0.1-5wt%, and is added to make consumers calm and alert.

Patent CN201280005750.7 discloses that cold-pressed and filtered cannabis juice can be mixed with raw materials such as fruit juice, vegetable juice and/or water, sugar and vanilla, and/or caffeine-containing ingredients to make soft drinks.

Patent US20170112161A1 discloses a method for producing a coffee product containing cannabinoid extracted from cannabis plants. The method includes extracting the cannabinoid from the cannabis plant and blending the cannabinoid into the coffee product.

In summary, although beverages containing the cannabinoids (such as the cannabidiol) and caffeine have been reported in the prior art, there is neither researches on the effect of CBD on ameliorating the anxiety behaviors induced by the caffeine, nor reports on the dose ratio of a composition containing the CBD and the caffeine.

Therefore, to overcome the shortcomings of the prior art, this application discloses a composition containing cannabidiol and caffeine, and application thereof.

### BRIEF SUMMARY OF THE INVENTION

It is surprisingly found in the invention that the combinational use of the cannabidiol or cannabis extract and the caffeine can effectively inhibit the anxiety behaviors induced by the caffeine.

The invention provides a composition, comprising (1) cannabidiol or a cannabis extract, and (2) caffeine. The cannabis extract contains the cannabidiol; and a mass ratio of the cannabidiol to the caffeine in the composition is (1-100):60.

Preferably, the mass ratio of the cannabidiol to the caffeine in the composition is (20-80):60. Further preferably, the mass ratio of the cannabidiol to the caffeine in the composition is (30-70):60. Most preferably, the mass ratio of the cannabidiol to the caffeine in the composition is 1:1.

Preferably, the cannabidiol is prepared by chemical synthesis, biosynthesis, plant extraction or other methods.

Preferably, a mass percentage of the cannabidiol in the cannabis extract is 10%-99%. For example, the cannabis extract contains 10%, 20%, 30%, 40%, 50%, 60%, 70%, 80%, 90%, 95%, and 99% of the cannabidiol in terms of mass percentage.

The cannabis extract of the invention is extracted from parts such as stalk cores, flowers, leaves, roots and/or seed shells of the cannabis. The cannabis extract according to the invention may be an extract or a combination of extracts separately extracted from different plant parts of the cannabis plants, or may be an extract or a combination of extracts simultaneously extracted from different plant parts or a combination thereof of the cannabis plants. Preferably, the parts for extraction are cannabis flowers and/or cannabis leaves.

The cannabis extract of the invention is extracted by conventional extraction methods in the pharmaceutical or food field, preferably by solvent extraction. An extracting solvent is selected from water, low-molecular-weight alcohol or an aqueous solution thereof, acetate, ketone, ether or low-boiling-point hydrocarbon; the low-molecular-weight alcohol includes methanol, ethanol, butanol or propanol; the acetate includes methyl acetate or ethyl acetate; the ketone includes acetone; the ether includes methyl ether or ether; and the low-boiling-point hydrocarbon includes aliphatic hydrocarbon, aromatic hydrocarbon or chlorinated hydrocarbon. Preferably, the extracting solvent is selected from water or an ethanol solution.

In a specific embodiment of the invention, the cannabis extract may be obtained by using a method as follows:
1) heating and refluxing raw materials using an extracting solvent or a mixture thereof;
2) filtering to remove residues;
3) performing liquid-liquid extraction;
4) regulating a pH value to 2-4;
5) extracting with the extracting solvent, and then removing the solvent; and
6) performing chromatographic separation to obtain a cannabis extract.

Preferably, in Step 1), the cannabis parts for extraction are heated for refluxing for at least 1 hour by using the extracting solvent or a mixture thereof 3-10 times the volume of the cannabis parts for extraction; in the method for extracting the cannabis extract, a solvent for the liquid-liquid extraction in Step 3) is a sodium hydroxide aqueous solution containing 20wt% of ethanol; the pH regulator in Step 4) is a mixture of a liquid-liquid extracted solution and a 5% sulfuric acid solution; and in the method for extracting the cannabis extract, a mobile phase mixture used in the chromatographic separation described in Step 6) consists of methanol/water and acetic acid or ethanol/water and acetic acid.

In a specific embodiment of the invention, the cannabis extract may be obtained by using a method as follows:
1) crushing raw materials to 10-40 meshes to obtain powder;
2) performing cold-soaked extraction on the obtained powder with an extraction solvent to obtain an extracting solution;
3) decolorizing the obtained extracting solution; and
4) concentrating to obtain the extractum of the cannabis extract.

Preferably, in Step 2), the cold-soaked extraction is performed 1-3 times, each for 0.5-2 hours, by using 30%-80% ethanol that is 2-8 times the volume of the powder; in Step 3), the decolorizing is performed by using 0.1-1wt % activated carbon for adsorbing decolorization; and the concentrating is performed at 70°C under reduced pressure or at room temperature to reach the relative density of 1.01-1.03.

The cannabis extract of the invention also includes one or more than one of cannabigerol CBG, cannabichromene, tetrahydrocannabinol THCV, and cannabigerol ester.

The invention also provides a composition, consisting of (1) a cannabis extract or cannabidiol, and (2) caffeine. The cannabis extract contains the cannabidiol, and a mass ratio of the cannabidiol to the caffeine in the composition is (1-100):60.

The composition of the invention may be a food composition or a pharmaceutical composition.

The invention also provides a pharmaceutical composition, comprising (1) cannabidiol or a cannabis extract, (2) caffeine and (3) a pharmaceutical adjuvant. The cannabis extract contains the cannabidiol, and a mass ratio of the cannabidiol to the caffeine in the pharmaceutical composition is (1-100):60. Preferably, the mass ratio of the cannabidiol to the caffeine in the pharmaceutical composition is (20-80):60. Further preferably, the mass ratio of the cannabidiol to the caffeine in the pharmaceutical composition is (30-70):60. Most preferably, the mass ratio of the cannabidiol to the caffeine in the pharmaceutical composition is 1:1.

Preferably, the mass percentage of the cannabidiol in the cannabis extract is 10%-99%. For example, the cannabis extract contains 10%, 20%, 30%, 40%, 50%, 60%, 70%, 80%, 90%, 95%, and 99% of the cannabidiol in terms of mass percentage.

Preferably, the pharmaceutical composition comprises 0.1%-1% of the cannabidiol, 0.06%-6% of the caffeine and 93%-99% of the pharmaceutical adjuvant in terms of mass percentage. Further preferably, the pharmaceutical composition comprises 0.1%-1% of the cannabidiol, 0.075%-1.5% of the caffeine and 97.5%-99% of the pharmaceutical adjuvant in terms of mass percentage. Most preferably, the pharmaceutical composition comprises 0.6% of the cannabidiol, 0.6% of the caffeine and 98.8% of the pharmaceutical adjuvant in terms of mass percentage.

Preferably, the pharmaceutical composition comprises 0.1%-10% of the cannabis extract, 0.06%-6% of the caffeine and 84%-99% of the pharmaceutical adjuvant in terms of mass percentage.

Preferably, the pharmaceutical adjuvant is one or a combination of two or more selected from a binder, a carrier, a pH regulator, a filler, a disintegrant, a flavoring agent, a stabilizer, a solubilizer, a dispersant, a film-forming agent, a plasticizer, a wetting agent, a solvent, a coating agent, a capsule shell, a colorant, a preservative, an antioxidant, a surfactant.

Preferably, the binder accounts for 2%-8% of the pharmaceutical composition in mass percentage; and the binder is one or a combination of two or more selected from cellulose, microcrystalline cellulose, hydroxypropyl cellulose, hydroxypropylmethyl cellulose, hydroxyethyl cellulose, ethyl cellulose, methyl cellulose, sodium hydroxymethyl cellulose, starch, micronized silica gel, starch slurry, syrup, maltose syrup, refined honey, polyvinylpyrrolidone, liquid glucose, cellulose derivatives, sodium alginate, polyethylene glycol, magnesium aluminum silicate, gelatin, and tragacanth. Further preferably, the binder accounts for 5% of the pharmaceutical composition in mass percentage; and the binder is one or a combination of two or more selected from microcrystalline cellulose, hydroxypropylmethyl cellulose, polyvinylpyrrolidone, and magnesium aluminum silicate.

Preferably, the pH regulator accounts for 0.1%-5% of the pharmaceutical composition in mass percentage, and is one or a combination of two or more selected from lactic acid, citric acid or a salt thereof, sodium bicarbonate, sodium hydroxide, ethanesulfonic acid, tartaric acid, aspart acid or a salt thereof, fumaric acid or a salt thereof, and methanesulfonic acid. Further preferably, the pH regulator is one or a combination of two or more selected from lactic acid, citric acid or a salt thereof, fumaric acid or a salt thereof; and the pH regulator accounts for 2% of the pharmaceutical composition in mass percentage.

Preferably, the filler is selected from microcrystalline cellulose, glucose, lactose, starch, fumed silica gel, sucrose, sodium carbonate, calcium carbonate, sorbitol, mannitol, dextrose, and propylene glycol; and the filler accounts for 4%-16% of the pharmaceutical composition in mass percentage. Further preferably, the filler is selected from starch and sorbitol; and the filler accounts for 13% of the pharmaceutical composition in mass percentage.

Preferably, the disintegrant is selected from alginic acid, agar, sodium bicarbonate, tartaric acid, citric acid, and sodium starch glycolate; and the disintegrant accounts for 4%-16% of the pharmaceutical composition in mass percentage. Further preferably, the disintegrant accounts for 10% of the pharmaceutical composition in mass percentage.

Preferably, the flavoring agent is one or a combination of two or more selected from lactose, glucose, mint, menthol, mint oil, stevioside, sucralose, xylitol, aspartame, saccharin sodium, trichlorosucrose, maple syrup, sorbitol, anethole, clove, lactitol, essence, maltose, and lemon oil; and the flavoring agent accounts for 0.02%-5% of the pharmaceutical composition in mass percentage. Further preferably, the flavoring agent is one or a combination of two or more selected from lactose, sodium saccharin, and trichlorosucrose; and the flavoring agent accounts for 3% of the pharmaceutical composition in mass percentage.

Preferably, the solvent is water or ethanol.

Preferably, the carrier is one or a combination of two or more selected from cyclodextrin or a derivative thereof, polyethylene glycol, microcrystalline cellulose, lactose, and polyvinylpyrrolidone; and the carrier accounts for 1%-16% of the pharmaceutical composition in mass percentage. Further preferably, the carrier is one or a combination of two or more selected from cyclodextrin or a derivative thereof, microcrystalline cellulose, and polyvinylpyrrolidone; and the carrier accounts for 9% of the pharmaceutical composition in mass percentage.

Preferably, the surfactant is one or a combination of two or more selected from sodium dodecyl sulfate, glycerin, sodium oleate, sodium lauroyl sarcosinate, and betaine; and the surfactant for 0.1%-5% of the pharmaceutical composition in mass percentage. Further preferably, the surfactant is sodium lauroyl sarcosinate or sodium dodecyl sulfate; and the surfactant accounts for 2.5% of the pharmaceutical composition in mass percentage.

Preferably, the preservative is one or a combination of two or more selected from benzoate, ascorbic acid, chlorphenesin or paraben. Preferably, the preservative accounts for 0.08%-0.15% of the total amount of the pharmaceutical composition in mass percentage. Preferably, the benzoate is sodium benzoate, potassium benzoate or calcium benzoate, and further preferably, is sodium benzoate. The paraben is methyl paraben, ethyl paraben, propyl paraben or sodium paraben mix, and further preferably, is methyl paraben. The preservative accounts for 0.12% of the total amount of the pharmaceutical composition in mass percentage.

Preferably, the lubricant is one or a combination of two or more selected from stearic acid, sodium stearate, calcium stearate, potassium stearate, polyethylene glycol, talc, and hydrogenated vegetable oil.

Preferably, the film-forming agent is one or a combination of two or more selected from sodium alginate, carrageenan, maltodextrin, chitosan, hydroxymethyl cellulose, hydroxyethyl cellulose, hydroxypropyl cellulose, trehalose, pectin, konjac gum, arabic gum, guar gum, pullulan, and locust bean gum. Preferably, the film-forming agent accounts for 70%-85% of the total amount of the pharmaceutical composition in mass percentage. More preferably, the film-forming agent accounts for 80%-85% of the total amount of the pharmaceutical composition in mass percentage. Preferably, the film-forming agent is sodium alginate.

Preferably, the plasticizer is one or a combination of two or more selected from glycerin, propylene glycol, sorbitol, mannitol, and ethylene glycol. Preferably, the plasticizer accounts for 10%-20% of the total amount of the pharmaceutical composition in mass percentage. More preferably, the plasticizer accounts for 10%-15% of the total amount of the pharmaceutical composition in mass percentage. Preferably, the plasticizer is glycerin or mannitol.

Preferably, the pharmaceutical composition may be in any conventional dosage forms, such as tablets, films, pulvis, capsules, granules, powders, pills, pigmentum for external use, plasters, solutions or injections.

The invention also provides a food composition, comprising (1) cannabidiol or a cannabis extract, and (2) caffeine. The cannabis extract contains the cannabidiol, and a mass ratio of the cannabidiol to the caffeine in the food composition is (1-100):60. Preferably, the mass ratio of the cannabidiol to the caffeine in the food composition is (20-80):60. Further preferably, the mass ratio of the cannabidiol to the caffeine in the food composition is (30-70):60. Most preferably, the mass ratio of the cannabidiol to the caffeine in the food composition is 1:1.

Preferably, a mass percentage of the cannabidiol in the cannabis extract is 10%-99%. For example, the cannabis extract contains 10%, 20%, 30%, 40%, 50%, 60%, 70%, 80%, 90%, 95%, and 99% of the cannabidiol in terms of mass percentage.

Preferably, the food may be a beverage, pasta, rice, edible oil, a dairy product, protein powder, porridge, soup, paste, meat, a soy product, chocolate, a candy or jelly.

Further preferably, the pasta is selected from flour, noodles, bread, fried dough twists, biscuits and dumplings. The rice is selected from rice flour. The edible oil is selected from blended oil. The milk product is selected from milk, yogurt and curd. The meat is selected from sausage. The soy product is selected from tofu and bean curd sticks.

Most preferably, the food composition is a beverage.

Preferably, the beverage in the invention further comprises a nutritional supplement substance. More preferably, the nutritional supplement substance has a mass percentage of 0.001%-70%. The nutritional supplement substance is one or a combination of two or more selected from a vitamin, a trace element, β-carotin, an amino acid, a γ-aminobutyric acid, taurine and a plant extract.

Preferably, the vitamin is one or a combination of two or more selected from vitamin B1, vitamin B2, niacin, vitamin B6, vitamin B12, vitamin C, vitamin E, and inositol; the amino acid is one or a combination of two or more selected from theanine, L-tyrosine, glycine, and glutamic acid; the trace element is one or a combination of two or more selected from iron, zinc, calcium, magnesium, copper, etc.; and the plant extract is one or a combination of two or more selected from a guarana extract, a black pepper extract, a maca extract, a ginseng extract, a cistanche extract, a kudzu root extract, a turmeric extract, a ginkgo extract, a wolfberry extract, an acai berry extract, a bilberry extract, a coconut extract, and a prunella vulgaris extract. Further preferably, the nutritional supplement substance is one or a combination of two or more selected from theanine, a guarana extract, L-tyrosine, γ-aminobutyric acid, taurine, a black pepper extract, vitamin C, vitamin E, β-carotene, niacin, vitamin B6, vitamin B12, and inositol.

Preferably, the beverage in the invention also contains an additive. More preferably, the additive in the beverage has a content of 0.1%-50% in mass percentage. The additive is one or a combination of two or more selected from spices, fruit and vegetable juice, scented tea juice, a colorant, an acidity regulator, a preservative, an emulsifier, an antioxidant, a thickener, a chelating agent, a stabilizer, and a sweetener.

Preferably, the fruit and vegetable juice is one or a combination of two or more selected from apple juice, grape juice, lychee juice, peach juice, longan juice, orange juice, pineapple juice, mulberry juice, pomegranate juice, carrot juice, watermelon juice, passion fruit juice, tomato juice, mango Juice, sugar cane juice, coconut juice, hami melon juice, rambutan juice, lemon juice, strawberry juice, passion fruit juice, tamarindus juice, basil juice, cocoa juice, pandanus juice, kiwi juice, white gourd juice, beetroot juice, blueberry juice, pear juice, cherry juice, betel nut juice, bitter gourd juice and plum juice and other edible juices. The flower tea juice according to the invention is one or a combination of two or more selected from green tea juice, roselle flower juice, jasmine tea juice, lemon grass juice, chrysanthemum juice, and safflower juice. The fruit and vegetable juice according to the invention may be original juice or concentrated juice. Further preferably, the fruit and vegetable juice according to the invention is one or a combination of two or more selected from concentrated cherry juice, concentrated apple juice, concentrated passion fruit juice, concentrated kiwi juice, and concentrated pear juice.

Preferably, the spice is phenethylamine.

Preferably, the sweetener is one or a combination of two or more selected from white sugar, high fructose corn syrup, sorbitol, aspartame, trichlorosucrose, xylitol, acesulfame potassium, maltitol, erythritol, isomaltitol, lactitol, lactose, maltose, mannose, trehalose, arabitol, saccharin, stevioside, maple syrup, and dulcin. Further preferably, the sweetener is one or a combination of two or more selected from high fructose corn syrup, sorbitol, aspartame, saccharin, and stevioside.

Preferably, the colorant is one or a combination of two or more selected from brilliant blue, sunset yellow, chlorophyll, sodium copper chlorophyllin, alluring red, iron oxide, amaranth red or carmine. Further preferably, the colorant is iron oxide or chlorophyll or a mixture thereof.

Preferably, the acidity regulator is one or a combination of two or more selected from citric acid or a salt thereof, malic acid or a salt thereof, fumaric acid or a salt thereof, adipic acid or a salt thereof, phosphoric acid or a salt thereof, gluconic acid or a salt thereof, tartaric acid or a salt thereof, ascorbic acid or a salt thereof, acetic acid or a salt thereof, phosphoric acid or a salt thereof. Further preferably, the acidity regulator is one or a combination of two or more selected from citric acid or a salt thereof, malic acid or a salt thereof, ascorbic acid or a salt thereof.

Preferably, the preservative is one or a combination of two or more selected from sodium benzoate, calcium benzoate, potassium benzoate, sodium sorbate, calcium sorbate, potassium sorbate, ascorbic acid, SHMP, BHA, BHT, TBHQ, dehydroacetic acid, dimethyl hydrogen carbonate, ethoxyquin, heptyl 4-hydroxybenzoate. Further preferably, the preservative is selected from sodium benzoate, sodium sorbate or heptyl 4-hydroxybenzoate.

Preferably, the chelating agent is selected from sodium acetate or EDTA.

Preferably, the stabilizer is one or a combination of two or more selected from modified starch, carrageenan, pectin, konjac gum, xanthan gum, sodium carboxymethyl cellulose, sodium alginate, sodium caseinate, and microcrystalline cellulose.

The beverage according to the invention may be a liquid beverage or a solid beverage.

The beverage according to the invention may contain CO₂ (a carbonated beverage), or may not contain CO₂ (a non-carbonated beverage). Preferably, when the beverage according to the invention is a carbonated beverage, the CO₂ gas capacity in the beverage is 2-2.5 times a beverage solvent (measured at 20°C).

The beverage according to the invention may be a non-alcoholic beverage, in which raw materials also include water.

The beverage according to the invention may be an alcoholic beverage, in which the alcohol content is 0.5%-60% (V/V), preferably 0.5%-10% (V/V). Then, the raw materials of the beverage also include one or a combination of two or more selected from alcohol, wine, yellow rice wine, beer, fruit wine, white wine, brandy, whiskey, vodka, rum, gin, milk wine, or other distilled wine.

The invention also provides a beverage preparation method, comprising the following steps.
a) Raw materials are weighed in proportion and placed in a container, and an appropriate amount of water is added to the container.
b) The container in Step a) is heated, and stirring and mixing are performed to obtain a mixture.
c) The mixed solution obtained in Step b) is subjected constant volume metering, filling, sterilizing, and cooling in sequence to obtain a beverage.

Preferably, the liquid added for reaching the constant volume in Step c) is one or a combination of two or more selected from water, alcohol, wine, yellow rice wine, beer, fruit wine, white wine, brandy, whiskey, vodka, rum, gin, milk wine or other distilled wine; the sterilizing is performed at 75°C-130°C.

Preferably, when the beverage is a carbonated beverage, the preparation method also includes filling CO₂ to the container.

The invention further provides application of cannabidiol or a cannabis extract in the preparation of a product for preventing and/or ameliorating adverse reactions of caffeine. Preferably, an active ingredient in the cannabis extract is cannabidiol.

Preferably, the preventing and/or ameliorating the adverse reactions induced by caffeine is preventing and/or ameliorating anxiety behaviors induced by the caffeine; and the product may be a medicine, food or a health product.

Unless otherwise specified, the description of "X times" in the invention means that the volume of the extraction solvent used is X times the mass of the parts for extraction. For example, for "3-10 times the amount", the amount of the extraction is 3 mL-10 mL if the mass of the cannabis parts for extraction is 1g.

The "ethanol" described in the invention is a mixture of pure ethanol and water or pure ethanol, wherein the concentration is in volume percentage (v/v%). For example, 30% ethanol as a mixture of ethanol and water contains 30% of pure ethanol and 70% of water, and 100% ethanol is absolute ethanol.

### DETAILED DESCRIPTION OF THE INVENTION

The technical solution in the embodiments of the present invention will be illustrated clearly and completely below. It is obvious that the embodiments described are merely some of the embodiments of the present invention, but not all of the embodiments. Based on the embodiments in the present invention, any other embodiments obtained by those ordinarily skilled in the art without making inventive efforts shall fall within the protection scope of the present invention.

### Embodiment 1 Preparation of Cannabis Extract

(1) Cannabis leaves used as raw materials were washed and air-dried.
(2) The air-dried raw materials described above were crushed and then sieved through a 40-mesh sieve.
(3) Resulting powder was cold-extracted with 45% ethanol 6 times the amount of the powder 2 times, for 1.5 hours each time;
(4) Extracting solutions were combined and then adsorbed with 0.5wt% activated carbon for decolourization.
(5) At the temperature of 70°C, the concentrating was performed under reduced pressure to reach a relative density of 1.02 to obtain the cannabis extract,

Among them, the mass content of the cannabidiol contained in the cannabis extract is 50%.

### Embodiment 2 Preparation of Cannabis Extract

(1) Cannabis leaves used as raw materials were washed and air-dried.
(2) The air-dried cannabis leaves from step (1) was heated for refluxing for 1 hour using 60% ethanol 9 times the amount of the cannabis leaves.
(3) The residues were removed by filtration and the solvent was removed under vacuum.
(4) The obtained extractum was heated at the temperature of about 125°C for about 40 minutes.
(5) Chromatographic separation was subsequently performed to obtain the cannabis extract, wherein a mobile phase mixture consists of ethanol/water and acetic acid.

Among them, the mass content of the cannabidiol contained in the cannabis extract is 90%.

### Embodiment 3 Preparation of Cannabis Extract

(1) Cannabis leaves used as raw materials were washed and air-dried.
(2) The air-dried cannabis leaves from Step (1) was heated for refluxing for 1.5 hour using 80% ethanol 7 times the amount of the cannabis leaves.
(3) Residues were removed by filtration.
(4) Liquid-liquid extraction was performed at least twice with 5wt% of sodium hydroxide aqueous solution. Among them, the sodium hydroxide aqueous solution contains 20wt% of ethanol.
(5) A liquid-liquid extracting solution was mixed with 5wt% of a sulfuric acid solution to regulate the pH value to about 3.
(6) Then, extraction was performed twice using aliphatic hydrocarbons, and the solvent was removed at low temperature under vacuum.
(7) Chromatographic separation was subsequently performed to obtain the cannabis extract, wherein a mobile phase mixture consists of ethanol/water and acetic acid.

Among them, the mass content of the cannabidiol contained in the cannabis extract is 99%.

### Embodiment 4 Preparation of Cannabis Extract

(1) Cannabis leaves used as raw materials were washed and air-dried.
(2) The air-dried raw materials described above were crushed and then sieved through a 10-mesh sieve.
(3) Resulting powder was cold-extracted 2 times with 30% ethanol 2 times the amount of the powder, for 1 hours each time;
(4) Extracting solutions were combined and then adsorbed with 0.5wt% activated carbon for decolourization.
(5) At room temperature, the concentration was performed under reduced pressure to reach a relative density of 1.02 to obtain the cannabis extract,

Among them, the mass content of the cannabidiol contained in the cannabis extract is 10%.

### Embodiment 5 Preparation of Pharmaceutical Composition Tablets

### I. Raw Materials (mass percentage)

0.2% of cannabis extract (obtained in Embodiment 1), 6% of caffeine, 84% of dextrin, 7% of sodium carboxymethyl starch, and 2.8% of magnesium stearate.

### II. Preparation Method

The cannabis extract and the caffeine were sieved and then mixed with dextrin and sodium carboxymethyl starch till a homogeneous mixture is formed. The mixture is then sieved and then mixed with magnesium stearate. Then, the resulting powder-type mixture is compressed into tablets of desired shape and size to obtain pharmaceutical tablets.

### Embodiment 6 Preparation of Pharmaceutical Composition Films

### I. Raw Materials (mass percentage)

0.66% of a cannabis extract (prepared in Embodiment 2), 0.36% of caffeine, 85% of maltodextrin, 10% of mannitol, 0.08% of sodium benzoate, 1.79% of trichlorosucrose, 0.11% of citric acid and 2% of microcrystalline cellulose.

### II. Preparation Method

a) The maltodextrin was swelled to obtain colloidal maltodextrin.
b) The cannabis extract, the caffeine, the sodium benzoate, the trichlorosucrose, the citric acid, the microcrystalline cellulose, and the mannitol were added to the colloidal maltodextrin obtained in Step a) to obtain a colloidal mixture.
c) The colloidal mixture obtained in Step b) was formed into a thin film by using a scraping method;
d) The film obtained in Step c) was dried to obtain a pharmaceutical film.

### Embodiment 7 Preparation of Pharmaceutical Composition Capsules

### I. Raw Materials (mass percentage)

2% of cannabis extract (obtained in Embodiment 3), 2% of caffeine, 42% of microcrystalline cellulose, 42% of pregelatinized starch, 10% of croscarmellose and 2% of magnesium stearate.

### II. Preparation Method

The cannabis extract and the caffeine were sieved and mixed with other adjuvants; and a resulting mixture was filled into glutoid capsules to obtain pharmaceutical capsules.

### Embodiment 8 Preparation of Pharmaceutical Composition Capsules

### I. Raw Materials (mass percentage)

2% of cannabis extract (obtained in Embodiment 4), 0.2% of caffeine, 42.9% of microcrystalline cellulose, 42.9% of pregelatinized starch, 10% of croscarmellose and 2% of magnesium stearate.

### II. Preparation Method

The cannabis extract and the caffeine were sieved and mixed with other adjuvants; and a resulting mixture was filled into glutoid capsules to obtain pharmaceutical capsules.

### Embodiment 9 Preparation of Beverage

### I. Raw Materials (mass percentage)

0.1% of cannabidiol (purity>99%), 0.06% of caffeine, 0.2% of γ-aminobutyric acid, 4% of guarana extract, 0.2% of theanine, 60% of L-tyrosine, 0.6% of taurine, 0.1% of vitamin E, 20% of concentrated apple juice, 0.4% of stevioside, 2% of chlorophyll, 0.14% of malic acid, and the balance of water.

### II. Preparation Method

a) Raw materials were weighed in proportion and placed in a container, and an appropriate amount of water was added to the container.
b) The container in Step a) was heated, and stirring and mixing was performed to obtain a mixture.
c) The mixed solution obtained in Step b) was subjected to volume metering to 100 mL, filling, sterilizing at 100°C, and cooling to obtain a beverage.

### Embodiment 10 Preparation of Beverage

### I. Raw Materials (mass percentage)

0.1% of cannabidiol (purity > 99%), 6% of caffeine, 0.05% of vitamin C, 0.1% of niacin, 40% of concentrated cherry juice, 3% of sorbitol, 0.4% of chlorophyll, 0.1% of iron oxide, 0.15 % of ascorbic acid and the balance of water.

### II. Preparation Method

a) Raw materials were weighed in proportion and placed in a container, and an appropriate amount of water was added to the container.
b) The container in Step a) was heated, and stirring and mixing was performed to obtain a mixture.
c) The mixed solution obtained in Step b) was added with water to reach a constant volume of 100 mL, filled, sterilized at 75°C, and then cooled to room temperature.
d) Carbon dioxide was filled so that the gas capacity of the product was 2.0 times (at 20°C) that of the beverage to obtain a beverage.

### Embodiment 11 Preparation of Beverage

### I. Raw Materials (mass percentage)

1% of cannabidiol (purity > 99%), 0.6% of caffeine, 0.15% of β-carotene, 0.08% of phenethylamine, 4% of aspartame, 0.1% of citric acid, 0.12% of calcium benzoate, and the balance of milk wine.

### II. Preparation Method

a) Raw materials were weighed in proportion and placed in a container, and an appropriate amount of water was added to the container.
b) The container in Step a) was heated, and stirring and mixing was performed to obtain a mixture.
c) The mixed solution obtained in Step b) was added with milk wine to reach a constant volume of 100 mL, filled, sterilized at 130°C, and cooled in sequence to obtain a beverage.

### Embodiment 12 Preparation of Beverage

### I. Raw Materials (mass percentage)

0.1% of cannabis extract (obtained in Embodiment 3), 0.2% of caffeine, 0.1% of vitamin E, 0.05% of inositol, 0.08% of glycine, 50% of L-tyrosine, 25% of grape juice, 0.08% of phenethylamine, 4% of sorbitol, 0.09% of amaranth red, 0.2% of malic acid, 0.1% of heptyl p-hydroxybenzoate, and the balance of water.

### II. Preparation Method

a) Raw materials were weighed in proportion and placed in a container, and an appropriate amount of water was added to the container.
b) The container in Step a) was heated, and stirring and mixing was performed to obtain a mixture.
c) The mixed solution obtained in Step b) was added with water to reach a constant volume of 100 mL, filled, sterilized at 130°C, and cooled in sequence to obtain a beverage.

### Embodiment 13 Preparation of Beverage

### I. Raw Materials (mass percentage)

10% of cannabis extract (obtained in Embodiment 4), 1% of caffeine, 0.15% of β-carotene, 0.09% of vitamin C, 0.08% of phenethylamine, 4% of guarana extract, 30% of mango juice, 3% of maltitol, 0.1% of sodium citrate, 0.12% of sodium benzoate, and the balance of water.

### II. Preparation Method

a) Raw materials were weighed in proportion and placed in a container, and an appropriate amount of water was added to the container.
b) The container in Step a) was heated, and stirring and mixing was performed to obtain a mixture.
c) The mixed solution obtained in Step b) was added with water to reach a constant volume of 100 mL, filled, sterilized at 130°C, and cooled in sequence to obtain a beverage.

### Embodiment 14 Amelioration of Anxiety Behaviors Induced by Caffeine with Cannabidiol (CBD)

### Test 1

### I. Objective

To study the effect of different doses of CBD on preventing and treating rat anxiety behavior induced by high-dose intake of caffeine.

### II. Test Animals and Groups

Test Animals: 50 SPF male wistar rats (180-200 g). Feeding Conditions: SPF animal houses, free feeding, 12 h light/12h darkness.

Test Groups: After one week of adaptive feeding, rats were randomly divided into 5 groups as follows according to body weights: a normal control group, a caffeine model group, a CBD-1mg/kg group, a CBD-10mg/kg group, and a CBD-100mg/kg group. Except for the normal control group, animals in other groups were intragastrically administrated with 60 mg/kg caffeine saline solution. Each CBD administration group was also intragastrically administrated with a corresponding dose of a CBD soybean oil solution at the same time, and then an elevated plus-maze test was conducted within 30 minutes after administration.

### III. Test Methodology

Elevated Plus-maze (EMP) Test: A maze was cross-shaped and consists of 2 open arms (OAs), two closed arms (CAs) and a central platform. The maze was fixed on a support, 50 cm above the ground. A camera was placed above the central platform. The behaviors of rats were recorded and analyzed through a computer. The test was conducted in a quiet shaded condition. Before the test, the rats to be tested were placed in one rat cage. After freely exploring for 5 minutes, the rats were quickly placed on the central platform of the EMP. The observation test was started immediately after release. Each rat was tested for five minutes. After each rat was tested, the maze was thoroughly cleaned with a towel dipped in clean water and low-concentration ethanol. After the ethanol volatilizes and diffuses, the test on the next rat began.

### Test Items:

(1) Open arm entry (OE): the number of entries into any open arm was based on the fact that four paws of each rat were located within the arms, and when any paw was completely withdrawn from the arms halfway, the current entry event was deemed as being completed.
(2) Open arm time (OT): it was measured in second.
(3) Closed arm entry (CE): the number of entries into any closed arm, which was determined based on the same standard as OE.
(4) Closed arm time (CT): it was measured in second.
(5) Calculation of the proportion of the number of entries into the open arm (OE%): namely, OE/(OE+CE)×100%.
(6) Proportion of residence time in the open arms (OT%): namely, OT/(OT + CT)×100%. OE% and OT% were used as indexes for evaluating the anxiety state of rats.

### Statistical Method:

The data was expressed with *x̅*±*s*. One-way analysis of variance (one way ANOVA) was used to compare multi-sample means, and t test was used for inter-group comparison.

### IV. Results

The number, time and ratio of the entries into the arms are shown in Table 1.

**Table 1 Number, time and ratio of entries into arms**

| | OE (times) | OT (s) | OE% | OT% | CE (times) | CT (s) |
|---|---|---|---|---|---|---|
| Normal Control | 5.3±0.48 | 105±4.52 | 52.94 | 34.4 | 4.8±0.68 | 200±1.24 |
| Caffeine Model | 2.1±0.78 | 42.9±1.89 | 21.21 | 14.50 | 7.8±0.11 | 253±5.09 |
| CBD-1 mg/kg | 3.2±0.45 | 58.1±3.30 | 31.37 | 19.24 | 7.0±0.92 | 244±5.14 |
| CBD-10mg/kg | 4.8±0.91 | 85±2.07 | 47.52 | 28.43 | 5.3±1.07 | 214±4.01 |
| CBD-100mg/kg | 5.4±0.25 | 100±1.32 | 54.00 | 33.22 | 4.5±0.91 | 201±3.05 |

From Table 1, it can be seen that CBD shows a significant effect on inhibiting the anxiety behavior of rats. The OE% of the caffeine model group is 21.21%; there is a large slope from CBD-1mg/kg to CBD-10mg/kg after the intragastric administration for the rats, exhibiting significant amelioration of the anxiety behavior; but the slope from CBD-10mg/kg to CBD-100mg/kg after intragagstric administration is slightly smaller than before, exhibiting certain amelioration of the anxiety behavior.

### Test 2

### I. Test Animals and Groups

Test Animals: 100 SPF male wistar rats (180-200 g). Feeding Conditions: SPF animal houses, free feeding, 12 h light/12h darkness.

Test Groups: After one week of adaptive feeding, rats were randomly divided into 10 groups as follows according to body weight: a normal control group, a group A (prepared in Embodiment 5), a group B (prepared in Embodiment 6), a group C (prepared in Embodiment 7), a group D (prepared in Embodiment 9), a group E (prepared in Embodiment 10), a group F (prepared in Embodiment 11), a group G (prepared in Embodiment 8), a group H (prepared in Embodiment 12) and a group I (prepared in Embodiment 13). Except for the normal control group (intragagstric administration with normal saline), all the administration groups were intragagstrically administrated with the pharmaceutical composition or 50 mL of beverage; an elevated plus-maze test was conducted within 30 min after the administration; and the rats were measured in blood pressure non-invasively after the behavioral test was ended.

### II. Test Methodology and Test Consistency

### III. Test Results

The number, time and ratio of the entries into the arms are shown in Table 2.

**Table 2 Results of the ratios of number of entries into the open arms and the ratios of time thereof**

| | Normal Control | A | B | C | D | E | F | G | H | I |
|---|---|---|---|---|---|---|---|---|---|---|
| OE% | 52.94 | 47.17 | 52.80 | 50.12 | 53.14 | 47.87 | 51.91 | 49.14 | 48.52 | 49.98 |
| OT% | 34.41 | 30.10 | 33.19 | 32.18 | 34.74 | 30.73 | 33.92 | 31.96 | 31.00 | 31.79 |

From Table 2, it can be seen that the ratio of the number of entries into the open arms and the ratios of time thereof for the rats administered with the products of Embodiments 5-13 (the groups A-I) are similar to those under normal conditions. That is, the rats basically have no anxiety behaviors compared with the normal control group. Meanwhile, the caffeine model groups show the OE% of 21.21% according to Experiment 1, showing obvious anxiety behaviors. That is to say, the administration of the pharmaceutical composition or beverage prepared in the embodiments of the invention will not induce anxiety behaviors. This partly demonstrates that the cannabis extract or the cannabidiol has a significant effect on inhibiting the anxiety behaviors induced by the caffeine in rats. In particular, the rats in groups B, F and D basically show no excessive anxiety behaviors, and the blood pressure of the rats in groups A-I were within the normal range after the test.

The preferred embodiments of the invention are described in detail above, but the invention is not limited to the specific details in the above embodiments. Various simple modifications can be made to the technical solutions of the invention within the scope of the technical idea of the invention. All of these simple modifications belong to the protection scope of the invention.

In addition, it should be noted that the specific technical features described in the above specific embodiments can be combined in any suitable manner without contradictions. To avoid unnecessary repetition, the invention will not make separate explanation for the various possible combinations.

## Claims

1. A composition, comprising (1) cannabidiol or a cannabis extract, and (2) caffeine, wherein the cannabis extract contains the cannabidiol, and a mass ratio of the cannabidiol to the caffeine in the composition is (1-100):60.

2. The composition according to Claim 1, wherein a mass percentage of the cannabidiol in the cannabis extract is 10%-99%.

3. The composition according to Claim 1 or 2, wherein the composition is a food composition or a pharmaceutical composition.

4. A pharmaceutical composition, comprising (1) cannabidiol or a cannabis extract, (2) caffeine and (3) a pharmaceutical adjuvant, wherein the cannabis extract contains the cannabidiol, and a mass ratio of the cannabidiol to the caffeine in the pharmaceutical composition is (1-100):60.

5. The pharmaceutical composition according to Claim 4, wherein a mass percentage of the cannabidiol in the cannabis extract is 10%-99%.

6. The pharmaceutical composition according to Claim 4 or 5, wherein the pharmaceutical composition comprises 0.1%-1% of the cannabidiol, 0.06%-6% of the caffeine and 93%-99% of the pharmaceutical adjuvant in terms of mass percentage.

7. The pharmaceutical composition according to any one of Claims 4-6, wherein the pharmaceutical adjuvant is one or a combination of two or more selected from a binder, a carrier, a pH regulator, a filler, a disintegrant, a flavoring agent, a stabilizer, a film-forming agent, a plasticizer, a solubilizer, a dispersant, a wetting agent, a solvent, a coating agent, a capsule shell, a colorant, a preservative, an antioxidant, a surfactant.

8. The pharmaceutical composition according to any one of Claims 4-7, wherein the pharmaceutical composition is tablets, pulvis, capsules, granules, powders, pills, pigmentum for external use, plasters, films, solutions or injections.

9. A food composition, comprising (1) cannabidiol or a cannabis extract, and (2) caffeine, wherein the cannabis extract contains the cannabidiol, and a mass ratio of the cannabidiol to the caffeine in the food composition is (1-100):60.

10. The food composition according to Claim 9, wherein a mass percentage of the cannabidiol in the cannabis extract is 10%-99%.

11. The food composition according to Claim 9 or 10, wherein the food is selected from a beverage, pasta, rice, edible oil, a dairy product, protein powder, porridge, soup, paste, meat, a soy product, chocolate, a candy or jelly.

12. The food composition according to Claim 11, wherein the food is the beverage which further comprises a nutritional supplement substance, and the nutritional supplement substance is one or a combination of two or more selected from a vitamin, a trace element, β-carotin, an amino acid, an γ-aminobutyric acid, taurine and a plant extract.

13. The food composition according to Claim 12, wherein the beverage further contains an additive, and the additive is one or a combination of two or more selected from spices, fruit and vegetable juice, scented tea juice, a colorant, an acidity regulator, a preservative, an emulsifier, an antioxidant, a thickener, a chelating agent, a stabilizer, and a sweetener.

14. The food composition according to Claim 13, wherein the beverage is a liquid beverage or a solid beverage.

15. The food composition according to Claim 13, wherein the beverage is a carbonated beverage or a non-carbonated beverage.

16. Application of cannabidiol or a cannabis extract in the preparation of a product for preventing and/or ameliorating adverse reactions of caffeine.

17. Application of the cannabidiol or cannabis extract according to Claim 16 in the preparation of a product for preventing and/or ameliorating adverse reactions of caffeine, wherein the preventing and/or ameliorating the adverse reactions of the caffeine is preventing and/or ameliorating anxiety behaviors induced by the caffeine.
